# EUROPÄISCHE PATENTSCHRIFT

(11) **EP 1 055 730 B1**
(45) Veröffentlichungstag und Bekanntmachung des Hinweises auf die Patenterteilung: **20.03.2002**
(21) Anmeldenummer: 00109843.3
(22) Anmeldetag: 10.05.2000
(51) Int. Cl.: C12N 15/52, C12N 15/77, C12P 13/08

(54) **Verfahren zur fermentativen Herstellung von L-Aminosäuren unter Verwendung coryneformer Bakterien**
Process for the fermentative production of L-amino acids employing coryneform bacteria
Procédé de production de L-aminoacides par fermentation de bactéries coryneformes

(30) Priorität: 27.05.1999 DE 19924364
(43) Veröffentlichungstag der Anmeldung: 29.11.2000
(73) Patentinhaber: Degussa AG, 40474 Düsseldorf (DE); FORSCHUNGSZENTRUM JÜLICH GMBH, 52425 Jülich (DE)
(72) Erfinder: Tilg, Yvonne, 40822 Mettmann (DE); Eikmanns, Bernhard, Prof. Dr., 89081 Ulm (DE); Eggeling, Lothar, Dr., 52428 Jülich (DE); Sahm, Hermann, Prof. Dr., 52428 Jülich (DE); Möckel, Bettina, Dr., 40597 Düsseldorf (DE); Pfefferle, Walter, Dr., 33790 Halle (DE)

(56) Entgegenhaltungen:
- EP-A- 0 358 940
- EP-A- 0 387 527
- EP-A- 0 435 132
- DE-A- 3 823 451
- US-A- 4 560 654
- JAEGER WOLGANG ET AL: "A Corynebacterium glutamicum gene encoding a two-domain protein similar to biotin carboxylases and biotin-carboxyl-carrier proteins." ARCHIVES OF MICROBIOLOGY, Bd. 166, Nr. 2, 1996, Seiten 76-82, XP000946309 ISSN: 0302-8933
- PEREZ CARLOS A ET AL: "Effects on Bacillus subtilis of conditional expression of the accBC operon encoding subunits of acetyl coenzyme A carboxylase, the first enzyme of fatty acid synthesis." MICROBIOLOGY (READING), Bd. 144, Nr. 4, April 1998 (1998-04), Seiten 895-903, XP000946310 ISSN: 1350-0872

## Beschreibung

Gegenstand der Erfindung ist ein Verfahren zur fermentativen Herstellung von L-Aminosäuren, insbesondere L-Lysin unter Verwendung von coryneformen Bakterien, in denen das accBC-Gen verstärkt wird.

### Stand der Technik

L-Aminosäuren, insbesondere L-Lysin finden in der Tierernährung, in der Humanmedizin und in der pharmazeutischen Industrie Anwendung.

Es ist bekannt, daß diese Aminosäuren durch Fermentation von Stämmen coryneformer Bakterien insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Aminosäuren sind und L-Aminosäuren produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Aminosäure-Biosynthesegene amplifiziert und die Auswirkung auf die L-Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6, 261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von L-Aminosäuren, insbesondere L-Lysin bereitzustellen.

### Beschreibung der Erfindung

L-Aminosäuren, insbesondere L-Lysin finden in der Tierernährung, in der Humanmedizin und in der pharmazeutischen Industrie Anwendung. Es besteht daher ein allgemeines Interesse daran neue, verbesserte Verfahren zur Herstellung dieser Verbindungen bereitzustellen.

Wenn im folgenden L-Lysin oder Lysin erwähnt wird, ist damit nicht nur die Base sondern es sind auch die Salze wie z. B. Lysin-Monohydrochlorid oder Lysin-Sulfat gemeint.

Gegenstand der Erfindung ist ein Verfahren zur fermentativen Herstellung von L-Aminosäuren, insbesondere L-Lysin unter Verwendung von coryneformen Bakterien, die insbesondere bereits die gewünschte Aminosäure produzieren und in denen die die Biotin-Carboxyl-Trägerprotein-Domäne und die Biotin-Carboxylase-Domäne tragende Untereinheit des Enzyms Acetyl-CoA Carboxylase codierende Nucleotidsequenz (accBC-Gen) verstärkt, insbesondere überexprimiert wird.

Bevorzugte Ausführungsformen finden sich in den Ansprüchen.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren, insbesondere L-Lysin aus Glucose, Saccharose, Lactose, Fructose, Maltose, Melasse, Stärke, Cellulose oder aus Glycerin und Ethanol herstellen. Es handelt sich um Vertreter coryneformer Bakterien, insbesondere der Gattung Corynebacterium. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren, insbesondere L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Brevibacterium flavum FERM-P 6463 und
Brevibacterium flavum FERM-P 6464.

Das accBC-Gen kodiert für eine Untereinheit der Acetyl-CoA Carboxylase, die eine Biotin-Carboxyl-Trägerprotein-Domäne und eine Biotin-Carboxylase-Domäne trägt. Die Nukleotidsequenz des accBC-Gens von Corynebacterium glutamicum wurde von Jäger et al. (Archives of Microbiology 166, 76 - 82 (1996)) bestimmt und ist bei der Datenbank der European Molecular Biology Laboratories (EMBL, Heidelberg, Deutschland) unter der Accession Number U35023 allgemein verfügbar.

Das von Jäger et al. (Archives of Microbiology 166, 76 - 82 (1996)) beschriebene accBC-Gen von C. glutamicum kann erfindungsgemäß verwendet werden. Weiterhin können Allele der accBC-Gene verwendet werden, die sich aus der Degeneriertheit des genetischen Codes oder durch funktionsneutrale Sinnmutationen (sense mutations) ergeben.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen L-Lysin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EP-B 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A- 10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

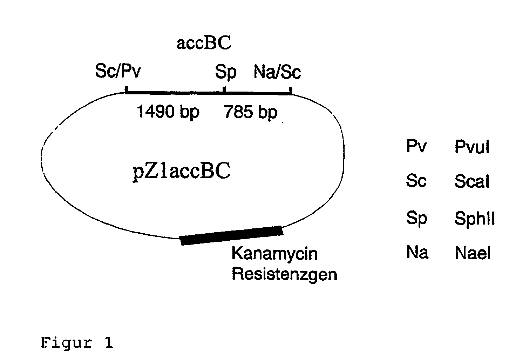

Ein Beispiel für ein Plasmid mit Hilfe dessen das accBC-Gen überexprimiert werden kann, ist pZ1accBC (Figur 1), das in dem Stamm MH20-22B/pZ1accBC enthalten ist. Plasmid pZlaccBC ist ein auf Plasmid pZ1 (Menkel et al., Applied and Environmental Microbiology 55(3), 684 - 688 (1989)) basierender E. coli - C. glutamicum Pendelvektor, der das accBC-Gen trägt.

Zusätzlich kann es für die Produktion der L-Aminosäuren vorteilhaft sein neben den accBC-Genen ein oder mehrere Enzyme des entsprechenden Biosyntheseweges zu überexprimieren. So kann beispielsweise bei der Herstellung von L-Lysin
- gleichzeitig das für die Dihydrodipicolinat-Synthase kodierende dapA-Gen überexprimiert werden (EP-B 0 197 335), oder
- gleichzeitig ein S-(2-Aminoethyl)-Cystein - Resistenz vermittelndes DNA-Fragment amplifiziert werden (EP-A 0 088 166).

Weiterhin kann es für die Produktion von L-Aminosäuren vorteilhaft sein, neben der Überexpression des accBC-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoff enthaltende Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum der gewünschten L-Aminosäure gebildet hat. Dieses Ziel wird normalerweise innerhalb von 10 Stunden bis 160 Stunden erreicht.

Die Analyse von L-Aminosäuren kann durch Anionenaustauschchromatographie mit anschließender Ninhydrin Derivatisierung erfolgen, so wie bei Spackman et al. (Analytical Chemistry, 30, (1958), 1190) beschrieben.

Der Corynebacterium glutamicum Stamm DSM5715/pZ1accBC wurde unter der Nummer DSM 12786 bei der Deutschen Sammlung von Mikroorganismen und Zellkulturen (Braunschweig, Deutschland) nach dem Budapester Vertrag hinterlegt.

Das erfindungsgemäße Verfahren dient zur fermentativen Herstellung von L-Aminosäuren insbesondere L-Asparaginsäure, L-Asparagin, L-Homoserin, L-Threonin, L-Isoleucin und L-Methionin mit coryneformen Bakterien, insbesondere der Herstellung von L-Lysin.

### Beispiele

Die vorliegende Erfindung wird im folgenden anhand von Ausführungsbeispielen näher erläutert.

Zu diesem Zweck wurden Versuche mit dem L-Lysin produzierenden Stamm DSM5715, (EP-B- 0 435 132) durchgeführt, in denen die Überlegenheit des beanspruchten Verfahrens demonstriert wird:

### Beispiel 1

### Herstellung des Expressionsplasmids pZ1accBC und des Stamms DSM5715/pZ1accBC

Zur Konstruktion des Expressionsplasmids pZ1accBC, wurde das accBC-Gen enthaltende Plasmid pWJ71 (Jäger et al., Archives of Microbiology (1996) 166:76-82) mit den Restriktionsenzymen PvuI und NaeI verdaut und anschließend mit Klenow-Polymerase und alkalischer Phosphatase behandelt. Durch präparative Isolation aus einem Agarosegel, welche wie bei Sambrook et al. (Molecular Cloning a Laboratory Manual (1989) Cold Spring Harbour Laboratories) beschrieben durchgeführt wurde, wurde das 2,1 kbp grosse accBC-Gen tragende DNA-Fragment isoliert Parallel zur Präparation des accBC-Gens, wurde das Plasmid pZ1 (Menkel et al., Applied and Environmental Microbiology 55(3), 684 - 688 (1989)) mit dem Restriktionsenzym ScaI verdaut, und anschliessend ebenfalls eine Behandlung mit Klenow-Polymerase und alkalischer Phosphatase durchgeführt. Das präparierte accBC-Gen und der wie zuvor beschrieben behandelte Vektor pZ1 wurden ligiert, und der Stamm DSM5715 wie bei Liebl et al. (FEMS Microbiology Letters 65, 299-304 (1989)) beschrieben mit dem Ligationsgemisch transformiert. Die Selektion der Transformanten erfolgte auf Hirn-Herz-Agar der Firma Merck (Darmstadt, Deutschland), der mit 50 mg/l Kanamycin supplementiert worden war. Eine ausgewählte Transformante wurde als Stamm DSM5715/pZ1accBC bezeichnet. Die Restriktionskarte des Expressionsplasmids pZ1accBC ist in Figur 1 dargestellt.

### Beispiel 2

### Herstellung von L-Lysin

Der Stamm DSM5715/pZ1accBC wurde in Komplettmedium CgIII (Kase & Nakayama, Agricultural and Biological Chemistry 36 (9) 1611- 1621 (1972)), das mit 50 µg/ml Kanamycin supplementiert worden war, vorkultiviert. Hierzu wurde 10 ml Medium CgIII, die in 100 ml Erlenmeyerkolben mit 4 Schikanen enthalten waren, mit einer Impföse des Stammes angeimpft und die Kultur für 16 Stunden bei 240 rpm und 30°C bebrütet.

Zur Inokulierung von 10 ml Produktionsmedium, das in 100ml Erlenmeyerkolben mit 4 Schikanen enthalten war, wurde die OD (660nm) der Vorkultur bestimmt. Die das Produktionsmedium enthaltende Hauptkultur wurde auf eine OD von 0,1 angeimpft. Als Produktionsmedium wurde das von Keilhauer et al., beschriebene Medium CgXII verwendet (Journal of Bacteriology 175: 5595 - 5603 (1993)). Es wurden 4% Glukose und 50 mg/l Kanamycinsulfat zugesetzt. Inkubiert wurden die Zellen bei 33°C, 250 rpm und 80% Luftfeuchte für 48 Stunden.

Bei dem Verfahren mit Stamm DSM5715 enthielten die entsprechenden Medien kein Kanamycin.

Anschließend wurde die optische Dichte bei 660nm und die Konzentration an gebildetem L-Lysin mit einem Aminosäureanalysator der Firma Eppendorf-BioTronik (Hamburg, Deutschland) durch Ionenaustauschchromatographie und Nachsäulenreaktion mit Ninhydrinandetektion bestimmt. In Tabelle 1 ist das Ergebnis des Versuches dargestellt.

**Tabelle 1**

| Stamm | OD | L-Lysin g/l |
|---|---|---|
| DSM5175 | 31,4 | 7,2 |
| DSM5715/pZ1accB | 27,6 | 9,6 |

Folgende Figuren sind beigefügt:
- Figur 1:: Karte des Plasmids pZ1accBC.

## Patentansprüche

1. Verfahren zur Herstellung von L-Lysin durch Fermentation coryneformer Bakterien,
**dadurch gekennzeichnet,**
**daß** man Bakterien einsetzt, in denen man die intrazelluläre Aktivität des accBC-Enzyms erhöht, insbesondere die für das accBC-Gen codierende oder diese tragende Nucleotidsequenz überexprimiert.

2. Verfahren gemäß Anspruch 1,
**dadurch gekennzeichnet,**
**daß** man einen mit einem Plasmidvektor transformierten Stamm einsetzt und der Plasmidvektor die für die accBC-Gen codierende Nucleotidsequenz trägt.

3. Verfahren gemäß Anspruch 2,
**dadurch gekennzeichnet,**
**daß** man mit dem Plasmidvektor pZ1accBC, hinterlegt in Corynebacterium glutamicum, unter der Nummer DSM 12786 und dargestellt in Figur 1, transformierte Bakterien einsetzt.

4. Verfahren gemäß einem oder mehreren der Ansprüche 1 bis 3,
**dadurch gekennzeichnet,**
**daß** man coryneforme Bakterien verwendet, die L-Lysin herstellen.

5. Verfahren gemäß Anspruch 4,
**dadurch gekennzeichnet,**
**daß** gleichzeitig das für die Dihydrodipicolinat-Synthase kodierende dapA-Gen überexprimiert wird.

6. Verfahren gemäß Anspruch 5,
**dadurch gekennzeichnet,**
**daß** gleichzeitig ein S-(2-Aminoethyl)-Cystein-Resistenz vermittelndes DNA-Fragment amplifiziert wird.

7. Verfahren zur fermentativen Herstellung von L-Lysin gemäß einem oder mehreren der vorhergehenden Ansprüche,
**dadurch gekennzeichnet,**
**daß** man folgende Schritte durchführt:
a) Fermentation der L-Lysin produzierenden Bakterien, in denen zumindest die intrazelluläre Aktivität des accBC Enzyms erhört wird, insbesondere die für das accBC-Gen codierende oder diese tragende Nukleotidsequenz überexprimiert wird.
b) Anreicherung des L-Lysins im Medium oder in den Zellen der Bakterien und
c) Isolieren des produzierten L-Lysins.

## Claims

1. A process for the preparation of L-lysine by fermentation of coryneform bacteria, **characterised in that** bacteria are used in which the intracellular activity of the accBC enzyme is increased, particularly the nucleotide sequence coding for or bearing the accBC gene is overexpressed.

2. A process according to claim 1, **characterised in that** a strain transformed with a plasmid vector is used and the plasmid vector bears the nucleotide sequence coding for the accBC gene.

3. A process according to claim 2, **characterised in that** bacteria transformed with the plasmid vector pZ1accBC deposited in Corynebacterium glutamicum under number DSM 12786 and shown in Figure 1, are used.

4. A process according to one or more of claims 1 to 3, **characterised in that** coryneform bacteria that produce L-lysine are used.

5. A process according to claim 4, **characterised in that**, at the same time, the dapA gene coding for dihydrodipicolinate synthase is overexpressed.

6. A process according to claim 5, **characterised in that**, at the same time, a DNA fragment mediating S-(2-aminoethyl) cysteine resistance is amplified.

7. A process for the fermentative preparation of L-lysine according to one or more of the preceding claims, **characterised in that** the following steps are carried out:
a) fermentation of the L-lysine-producing bacteria in which at least the intracellular activity of the accBC enzyme is increased, particularly the nucleotide sequence coding for or bearing the accBC gene is overexpressed,
b) enrichment of the L-lysine in the medium or in the cells of the bacteria, and
c) isolation of the L-lysine produced.

## Revendications

1. Procédé pour la production de L-lysine par fermentation de bactéries corynéformes, **caractérisé en ce qu'**on utilise des bactéries dans lesquelles on augmente l'activité intracellulaire de l'enzyme accBC, en particulier **en ce qu'**on surexprime la séquence de nucléotides codant pour le gène accBC ou portant celui-ci.

2. Procédé selon la revendication 1, **caractérisé en ce qu'**on utilise une souche transformée par un vecteur de plasmide et **en ce que** le vecteur de plasmide porte la séquence de nucléotide codant pour le gène accBC.

3. Procédé selon la revendication 2, **caractérisé en ce qu'**on utilise des bactéries transformées avec le vecteur de plasmide pZ1accBC, déposé dans Corynebacterium glutamicum, sous le numéro DSM 12786 et représenté dans la figure 1.

4. Procédé selon l'une ou plusieurs des revendications 1 à 3, **caractérisé en ce qu'**on utilise des bactéries corynéformes qui produisent de la L-lysine.

5. Procédé selon la revendication 4, **caractérisé** en ce la dihydrodipicolinate-synthase codant pour le gène dapA est surexprimée simultanément.

6. Procédé selon la revendication 5, **caractérisé en ce qu'**un fragment d'ADN conférant une résistance à la S-(2-aminoéthyl)-cystéine est amplifié simultanément.

7. Procédé pour la production par fermentation de L-lysine selon l'une ou plusieurs des revendications précédentes, **caractérisé en ce qu'**on réalise les étapes suivantes:
a) fermentation des bactéries produisant de la L-lysine dans lesquelles on augmente au moins l'activité intracellulaire de l'enzyme accBC, en particulier **en ce qu'**on surexprime la séquence de nucléotide codant pour le gène accBC ou portant celui-ci,
b) enrichissement de la L-lysine dans le milieu ou dans les cellules des bactéries et
c) isolation de la L-lysine produite.
